Europäisches Patentamt

(19) European Patent Office

Office européen des brevets

(11) **EP 0 499 143 B1**

(12) **EUROPEAN PATENT SPECIFICATION**

(45) Date of publication and mention
of the grant of the patent:
**06.05.1999 Bulletin 1999/18**

(51) Int. Cl.$^6$: **A61K 31/60**

(21) Application number: **92101977.4**

(22) Date of filing: **06.02.1992**

(54) **Anti-reactive anti-asthmatic activity of acetylsalicylic acid by inhalation**

Antireaktive antiasthmatische Wirkung von Acetylsalicylsäure durch Inhalation

Activité anti-réactive anti-asthmatique de l'acide acetylsalicylique par voie d'inhalation

(84) Designated Contracting States:
**AT BE CH DE DK ES FR GB GR IT LI LU NL SE**

(30) Priority: **09.02.1991 EP 91101818**

(43) Date of publication of application:
**19.08.1992 Bulletin 1992/34**

(60) Divisional application:
**98110805.3**

(73) Proprietor:
**B.S.D. BIO SCIENCE DEVELOPMENT SNC Di
OMINI C. & ZUCCARI G.
20060 Bussero (Milano) (IT)**

(72) Inventor:
**Bianco, Sebastiano, Prof.
Milano (IT)**

(74) Representative:
**Bianchetti, Giuseppe, Prof. et al
Bianchetti Bracco Minoja S.r.l.
Via Rossini, 8
20122 Milano (IT)**

(56) References cited:
- L. OFFERHAUS et al.: "Farmacotherapie", 1989, pages 177-179, Amsterdam, NL; J. KREUKNIET: "Inhalatiecorticosteroiden bij astma"
- PULMONARY PHARMACOLOGY, vol. 1, no. 4, 1989, pages 187-191; M. ROBUSCHI et al.: "Inhaled frusemide is highly effective in preventing ultrasonically nebulised water bronchoconstriction"
- PROSTAGLANDINS LEUKOTRIENES AND MEDICINE, vol. 8, no. 3, March 1982, pages 239-248, Edinburgh, GB; A.J. FAIRFAX: "Inhibition of the late asthmatic responce to house dust mite by non-steroidal anti-inflammatory drugs"
- IDEM
- CHEST, vol. 80, no. 2, July-December 1981, pages 167-173, US; H. GONG, Jr., et al.: "Alcohol-induced bronchospasm in an asthmatic patient: pharmacologic evaluation of the mechanism"
- IDEM
- PROGRESS IN BIOCHEMICAL PHARMACOLOGY, vol. 20, 1985, pages 132-142, Basel, CH; S. BIANCO et al.: "Bronchial response to nonsteroidal anti-inflammatory drugs in asthmatic patients"
- THE JOURNAL OF ALLERGY AND CLINICAL IMMUNOLOGY, Allergy abstracts, index issue., vol. 71, no. 6, 1983, pages 560-567, US; J.T. CHIU: "Improvement in aspirin-sensitive asthmatic subjects after rapid aspirin desensitization and aspirin maintenance (ADAM) treatment"
- IDEM

- EUROPEAN JOURNAL OF RESPIRATORY DISEASES, vol. 69, no. 4, 1986, pages 219-225, Copenhagen, DK; M.L. KOWALSKI: "Clinical efficacy of aspirin in "desensitised" aspirin-sensitive asthmatics"
- IDEM
- BRITISH JOURNAL OF CLINICAL PHARMACOLOGY, vol. 10, Suppl. 2, 1980, pages 401S-405S, Macmillan Publ. Ltd, GB; A. SZCZEKLIK: "Analgesics, Allergy and Asthma",
- CLINICAL AND EXPERIMENTAL IMMUNOLOGY, vol. 52, no. 2, May 1983, pages 393-398, BlackWell Scientific Publ., OXFORD, GB; A.J. FAIRFAX et al.: "The late reaction following bronchial provocation with house dust mite allergen. Dependence on arachidonic acid metabolism"
- CURRENT THERAPEUTICS, vol. 24, no. 9, September 1983, pages 11-19, AU; A. SZCZEKLIK et al.: "Asthma and anti-inflammatory drugs: mechanisms and clinical patterns"
- DRUGS, vol. 32, Suppl. 4, 1986, pages 148-163, ADIS Press Ltd, AU; A. SZCZEKLIK: "Analgesics, Allergy and Asthma"
- JOURNAL OF ASTHMA, vol. 20, Suppl. 1, 1983, pages 23-29, Marcel Dekker, Inc. NEW YORK, US; A. SZCZEKLIK: "Anti-cyclo-oxygenase agents and asthma"
- GIORNALE ITALIANO DELLE MALATTIE DEL TORACE, vol. 40, no.1, 1986, pages 17-23, MILANO, IT; M. ROBUSCHI et al.: "Tollerabilita del tenoxicam negli astmatici aspirino-sensibili"
- UGESKRIFT FOR LAEGER, vol. 141, no. 17, 23rd April 1979, pages 1151-1152, GEA, KOBENHAVN, DK; F. EGEDE: "Fatalt forlobende astmatisk reaktion efter ketoprofen (Alreumat),(Orudis)

## Description

The invention relates to the use of the non-steroidal anti-inflammatory drugs acetylsalicylic acid for the production of an anti-reactive anti-asthmatic drug to be administered by inhalation; it relates also to this non-steroidal anti-inflammatory drug as a medicament to be inhaled for combating and preventing asthma.

Bronchial inflammation plays a key role in the current views on the pathogenetic mechanisms of bronchial asthma (Am. Rev. Respir. Dis. 1987; 136:740-51). Despite relatively large experimental evidence for the involvement of arachidonic acid metabolites in these mechanisms (J. Appl. Physiol. 1989, 66:578-583), several previous attempts to influence the bronchial responses using anti-inflammatory drugs with cyclooxygenase inhibitory activity have failed to show a clinically significant and consistent effect of these drugs in asthma (J. Allergy Clin. Immunol. 1983, 71:245-249). One of the limiting factors of the effect of these drugs on bronchial responses might be a scarce availability of these drugs in the lung following systemic administration. Indeed, a significant reduction of the bronchial response to ultrasonically nebulized water (UNW) in asthmatics after oral administration of relatively high doses of aspirin was observed (Progress in Biochemical Pharmacology (Karger, Basel) 1985, 20:132-142.2, and Allergologie, deuxieme edition, J. Charpin ed, Flammanion publ., Paris 1986, p. 683-693, and Respiration 1988, 54 (supp. 1):100-107).

Since a similar dose of the chemically related compound, sodium salicylate, which is devoid of inhibitory activity on cyclooxygenase, has no effect (L'acetilsalicilato di lisina ma non il salicilato protegge dal broncospasmo da $H_2O$. III Conferenza Italiana di Medicina Respiratoria, Milano 1990, Abstract p. 181), it can be concluded that cyclooxygenase inhibitors play a role in the therapeutic control of asthma, but relatively high doses have to be administered to afford a significant protective effect. Unfortunately, chronic treatment with such oral doses of NSAIDs causes an unacceptable rate of side effects, as indicated by two controlled studies available on the effect of treatment with high doses (1.3 to 2.6 g daily) of aspirin in asthmatics (J. Allergy. Clin. Immunol. 1984, 73:500-507; and J. Allergy Clin. Immunol. 1990, 85:59-65).

The aim of this invention was to make a breakthrough in the treatment of asthma with acceptable doses of a non-steroidal medicament.

This was accomplished by inhaling the non-steroidal anti-inflammatory drug (NSAID), acetylsalicylic acid (ASA).

The studies of this invention clearly show that inhaled NSAIDs, particularly lysine-acetylsalicylate (L-ASA), the soluble form of ASA, induce a remarkable attenuation of the bronchial responses to both non-specific (UNW) and specific (immediate and late reactions to allergen) stimuli in asthmatics in the absence of side-effects.

Suitable pharmaceutical compositions for inhalation are aerosols with a particle size of from 0.5 $\mu$m to 7 $\mu$m which enter the compartments of the lungs. Aqueous or aqueous-organic solutions or suspensions which can be nebulized in combination with propelling agents, such as fluoro chloro hydrocarbons, fluorinated hydrocarbons, dimethyl ether, propane, butane, nitrogen, carbon dioxide, $N_2O$, or mixtures of powders with microfine drugs, which can be administered using inhalers are particularly suitable. These pharmaceutical compositions may comprise appropriate additives, such as surface active substances, for instance phospholipids, sorbitane esters, polyoxysorbitane esters, or oleic acids, alcohols and polyols such as ethanol, glycerol, poly ethylene glycol, glucose, mannitol, sorbitol, in order to improve their relevant pharmaceutical properties.

## PATIENTS AND METHODS

**A)** Studies on non specific bronchial hyperreactivity:

Patients:

Clinically stable patients with either allergic or non-allergic asthma with a baseline forced expiratory volume at 1 second ($FEV_1$) greater than 70% of predicted, who were free of viral or bacterial respiratory infection for at least 4 weeks were studied. None of the patients had a history of L-ASA induced asthma. All the patients were either treated with inhaled beta2-stimulants or with inhaled beta2-stimulants and topical steroids, that were withheld for 10 hours before administration of the test drug (J. Allergy Immunol. 1975; 56:323-327).

Methods:

Bronchial reactivity to ultrasonically nebulized water (UNW) was measured as previously described (Eur. J. Respir. Dis. 1980 (suppl. 106):41-9; and Pulmonary Pharmacology 1989, 1:187-191). Each subject inhaled increasing doubling doses of UNW produced by an ultrasonic nebulizer (DeVilbiss Ultra-neb 99) set to an output of 2 ml/min. The subjects were instructed to breath a tidal volume, keeping the mouthpiece between their teeth with the mouth semi-opened. Doubling doses of UNW were administered by progressively increasing the time of exposure from 30″ (1 ml) to 240″ (15 ml) and if necessary for further 240″ setting the output to 4 ml/min (31 ml). Respiratory function was monitored after each dose, until a $FEV_1$ decrease of 20% or greater compared to baseline was observed. In the cases where specific airway resistance (sRaw) was measured, this was done during normal breathing using a constant-volume body plethysmograph with a closed bag system to condition air to body temperature pressure saturation (BTPS) (Fenyves & Gut, Basel, Switzerland) before and after pretreatment, and immediately after each test. $FEV_1$, was measured using a spirometer (Vitalograph). The best value of the first 3 technically satisfactory spirograms was chosen for analysis. The dose of UNW causing a 20% $FEV_1$ decrease ($PD_{20}$) was then calculated by interpolation on the cumulative dose-response curve.

Study design:

To test the effect of L-ASA on UNW-induced bronchoconstriction, in a group of 7 subjects with a positive response to a preliminary UNW challenge, the test was repeated twice, with intervals of 3-7 days between treatments which were either 5 ml of L-ASA (180 mg/ml in saline corresponding to 100 mg/ml of ASA) or placebo, according to a randomized protocol, by means of a nebulizer set to run to dryness in about 30′. The UNW challenge was repeated 30′ after treatment and once more the next day, 24 hours after treatment. The clinical characeteristics of the patients in this group are reported in Table I.

**B)** Allergen specific bronchial challenge:

Patients:

Volunteers with allergic asthma who had an early obstructive response after specific allergen bronchial challenge were recruited among patients attending an allergy clinic. All the patients had a clinical history of allergic asthma and/or rhinitis, showed a positive immediate skin reaction to the clinically relevant allergen, were either asymptomatic or had very mild respiratory symptoms, had a baseline $FEV_1$ over 70% of predicted and had been free of respiratory infections for a least 4 weeks. All the patients were either untreated or under occasional topical therapy, that was withheld as described for UNW test. Patients allergic to pollen were investigated outside the pollen season.

Methods:

Specific bronchial challenge was performed as reported in N. Engl. J. Med. 1989, 321: 1069-1073.

In a preliminary bronchial challenge, the allergen (Frazioni Alfa, Dome/Hollister-Stier, Bayropharm. Italiana, Milano, Italy) was administered by a dosimeter (MEFAR, Bovezza, Italy). The apparaturs was manually operated by the investigator and was set to deliver 3.7 µl/puff in 0.6 seconds, with a pause of 6 seconds between puffs.

The allergen was dissolved in normal saline at a concentration of 40 Activity Units (AU) per ml for doses up to 2.4 AU (corresponding to a delivered dose of 0.15 AU/puff), or 160 AU/ml for doses up to 9.6 AU (0.6 AU/puff), or 320 AU/ml (1.2 AU/puff) for higher doses. AU were determined by the manufacturer using the Radio Allergy Sorbant Test (RAST) inhibition assay compared with a reference preparation characterized by skin bioreactivity. $FEV_1$ and Peak Expiratory Flow Rate (PEFR) were measured using a dry spirometer (Vitalograph, Buckingham, England) and a mini-Wright Peak Flow Meter (Clement Clarke International Ltd., London, England) before and 10 minutes after administration of a first dose of 0.15 AU. The procedure was then repeated by doubling the allergen dose until a 25% or greater decrease of $FEV_1$ from baseline was observed, or a maximum allergen dose of 19.2 AU was reached. The provocative dose of allergen causing a 25% $FEV_1$ decrease ($PD_{25}$) was then calculated by interpolation from the cumulative dose-response curve, plotted on semilogarithmic paper.

Study design:

The effect of pre-treatment with inhaled NSAID on the bronchial responses to allergen challenge was investigated in three different studies.

In a first study, the effect of pre-treatment with L-ASA on the immediate bronchial allergic response was investigated in a double blind, cross-over study compared to placebo, using a random-number table for randomization.

Clinical characteristics of the patients selected for the study according to the above criteria are listed in Table I.

Each patient performed two bronchial challenges within an interval of 4 to 14 days, using a single dose of allergen corresponding to the $PD_{25}$ determined in the preliminary challenge. Before each test, the patients either received an aerosol with 4 ml of L-ASA 180 mg/ml in saline, corresponding to 100 mg/ml of acetyl salicylic acid (Flectadol[R], Maggioni-Winthrop S.p.A., Milan, Italy) or a placebo (normal saline) in 15 minutes, given by means of a jet nebulizer (mod. Soffio, Markos, Monza, Italy) set to an output of 0.27 ml/min. Immediately thereafter, the selected dose of allergen was delivered by a dosimeter. Specific airway resistance (sRaw) was measured during normal breathing using a constant-volume body plethysmograph with a closed bag system to condition air to BTPS (Fenyves & Gut, Basel, Switzerland) before and after pretreatment, and at 5, 10, 15, 20, 30, 45 and 60 minutes after the challenge. These measurements were made at least in quintuplicate and the mean was computed. $FEV_1$ was obtained by integration of flows measured with a No. 3 Fleish pneumotachograph connected to the body plethysmograph. The best value of the first 3 technically satisfactory spirograms was chosen for analysis.

To investigate the effect of pre-treatment with inhaled L-ASA on the late phase of the asthmatic reaction, two more patients presenting a dual asthmatic response to the preliminary challenge and characterized by the occurrence of a second obstructive response after the resolution of the immediate response, between the fourth and the eighth hour after challenge were studied. The study was conducted according to the previous protocol, except that respiratory function was monitored every 60 minutes up to eight hours after challenge.

Data analysis:

Data were expressed as absolute values or as a percentage of baseline values at time zero, i.e. after treatment with the active drug or placebo and immediately before challenge.

Changes of UNW reactivity were measured as doubling doses of UNW $PD_{20}$ compared to placebo, and calculated as

$$\log_2 (PD_{20} \text{ after drug}) - \log_2 (PD_{20} \text{ after placebo}).$$

An increase of the $PD_{20}$ by one doubling dose then correspond to a 100% increase of the cumulative dose of nebulized water causing a 20% $FEV_1$ decrease.

The percentage protective effects on the specific asthmatic response for $FEV_1$ and for sRaw were calculated for each patient according to the formula:

$$((\text{AUC placebo} - \text{AUC treatment}) / \text{AUC placebo}) \times 100,$$

where AUC is the area under the time-response curve of the absolute differences from baseline. All data were calculated without knowledge of the randomized treatment.

A two-way analysis of variance and a paired Student's test were used for statistical comparison of normally distributed variables, and the method of the least-significant difference was used for multiple comparisons (see Snedecor and Cochran, Statistical methods. 7th ed. Ames, Iowa University Press., 1980).

A level of p of less than 0.05 (two-tailed) was considered significant.

**RESULTS**

Studies on non-specific bronchial reactivity:

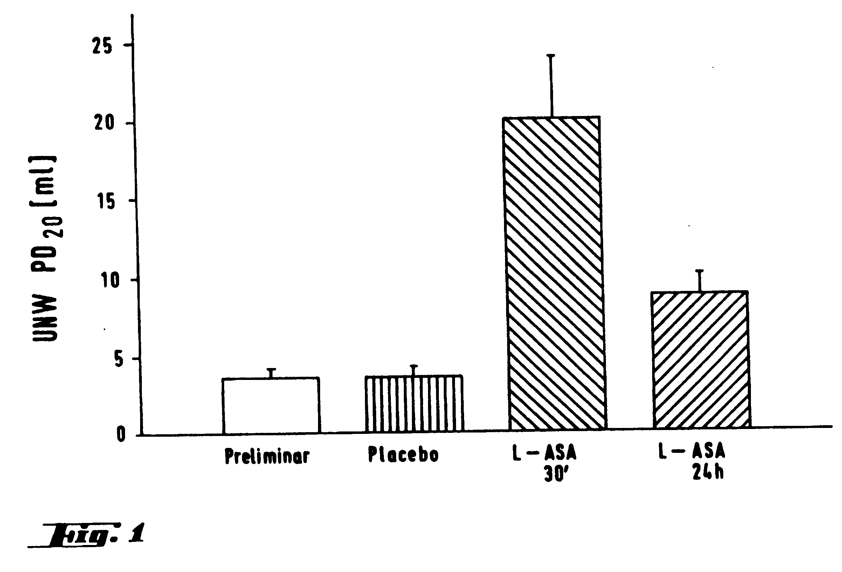

The effect of pre-treatment with L-ASA on UNW-induced bronchoconstriction is shown in Figure 1. The treatment was well tolerated, and no significant changes of baseline respiratory function parameters were observed on the different study days. The bronchial response to UNW after treatment with placebo was similar to the preliminary day. By contrast the UNW $PD_{20}$ after inhaled L-ASA was markedly increased by $2.42 \pm 0.20$ doubling doses ($M \pm SE$), and continued to be significantly increased compared to placebo even after 24 hours ($1.33 \pm 0.16$ doubling doses).

Studies on allergen challenge:

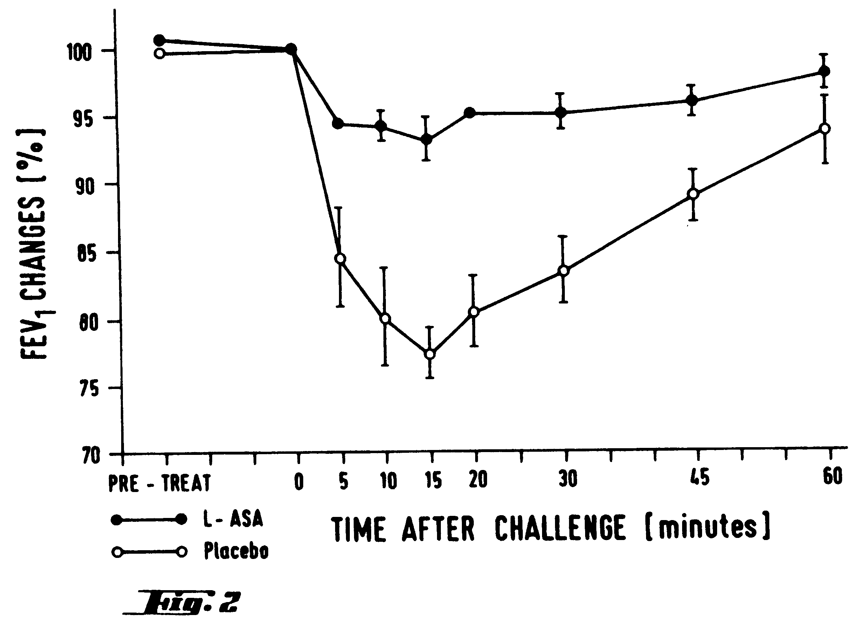

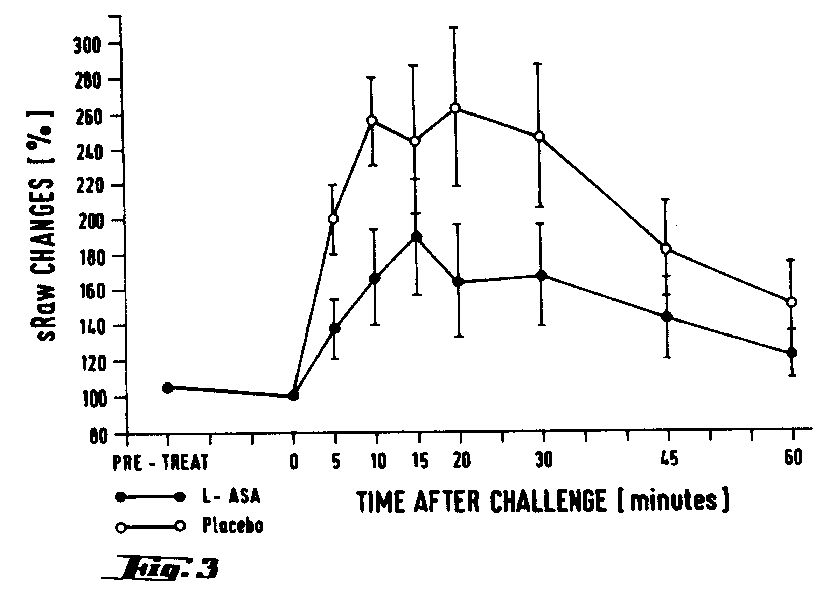

In a first study the effect of pre-treatment with inhaled L-ASA on the bronchial responses to allergen challenge was investigated in 6 patients. Baseline $FEV_1$ on the study days remained similar to those recorded during the preliminary challenge test. Values of $FEV_1$ and sRaw before and immediately after treatment on the study days were also not significantly different. After treatment with placebo, all the patients showed an immediate response which was characterized by a maximum decrease in $FEV_1$ of $24.5 \pm 2.3\%$ and a maximum increase in sRaw of $291 \pm 35\%$ of baseline values (Fig. 2 and 3). After treatment with inhaled L-ASA, the bronchial response was markedly reduced, and the mean post-challenge $FEV_1$ changes were significantly lower at every time point when compared with placebo (Figure 2). The effect on sRaw was somewhat less remarkable, probably due to the greater variability of the response. However, a remarkable difference was seen at 5 and 10 minutes compared to placebo (Figure 3). The maximum percentage decrease in $FEV_1$ and increase in sRaw ($8.6 \pm 1.0$ and $195 \pm 31\%$ of baseline, respectively) were also significantly smaller when as compared with placebo.

The protective activity of L-ASA on the immediate phase of the dual bronchial reaction, between 0 and 60 minutes, was $68 \pm 6\%$ when calculated from $FEV_1$ changes, and $56 \pm 18\%$ when calculated from the changes of sRaw. None of the patients had clinical manifestations of a late asthmatic response after any of the treatments.

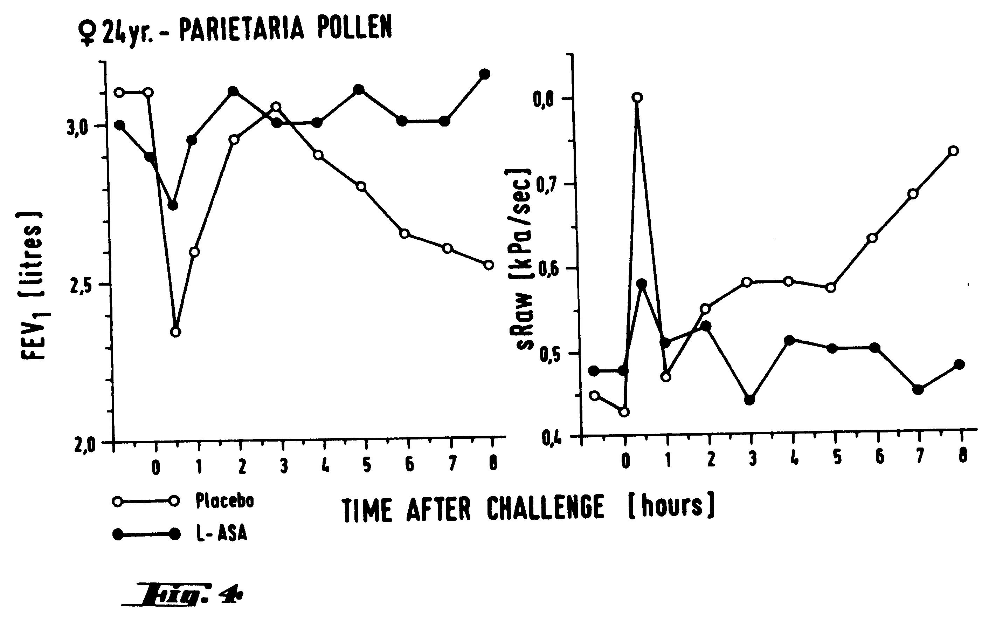

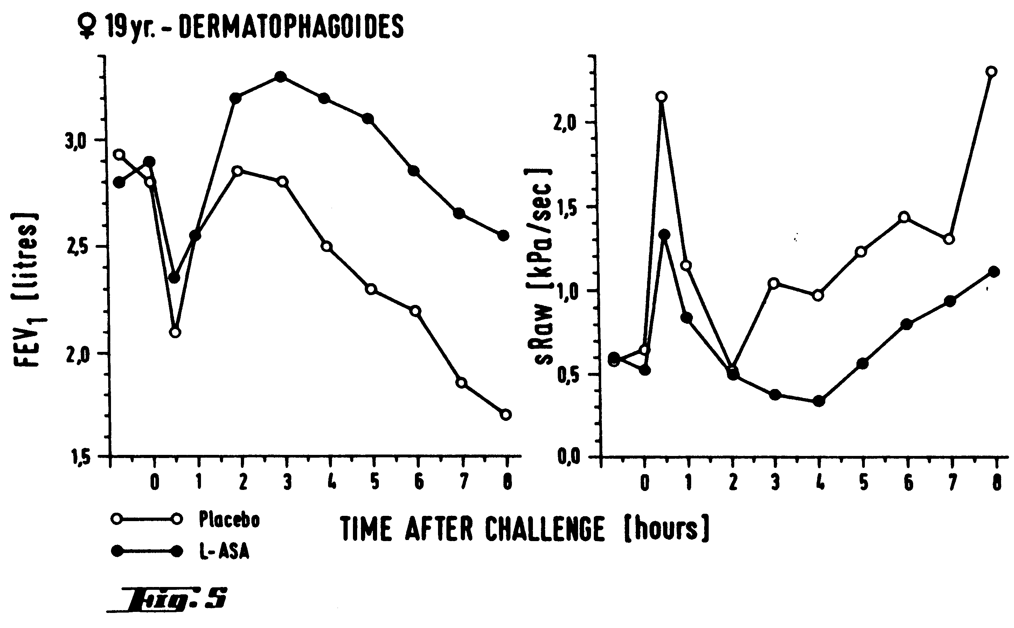

None of the subjects included in the studies above had a late asthmatic reaction to allergen challenge. To evaluate the effect of inhaled NSAIDs on this phenomenon, two other patients showing a dual asthmatic reaction after the preliminary challenge were selected. The effect of pre-treatment with L-ASA or placebo on the allergen-induced reaction in these subjects is shown in figures 4 and 5. In both subjects the late phase of the reaction was markedly attenuated after treatment with inhaled L-ASA.

Discussion

These data clearly show that theses problems of high oral doses, resulting in systemic side-effects, can be overcome by administration of ASA by inhalation. In fact, they indicate that the inhalation of ASA is able to inhibit the bronchial response to non-specific and specific bronchial stimuli in asthmatics. The inhalatory route is effective not only in reducing the side-effects in other organs which are bypassed by the topical treatment, but also in increasing the local activity of the drugs. The inhalatory route also takes advantage of a delayed drug metabolism, revealing the surprisingly long-lasting effect of inhaled L-ASA on UNW induced bronchospasm (Figure 1).

Finally, it is of particular interest that we have observed a good protective effect of inhaled L-ASA also on the late phase of the asthmatic response to allergen (Figures 4 and 5).

**FIGURE LEGENDS**

Figure 1. Effect of inhaled L-ASA (100 mg/ml) on bronchial reactivity to UNW at 30′ and 24 hrs after treatment. Data are expressed as $M \pm SE$ of UNW $PD_{20}$. Clinical characteristics of the study group are reported in Table I.

Figure 2. Time course of $FEV_1$ changes, expressed as percentage of post-treatment value (time 0), after specific allergen challenge. Bars represent mean and SE after inhalation of placebo (unfilled circles and L-ASA (filled circles). Baseline values (arrow) were $3.90 \pm 0.2$ l ($M \pm SE$) before placebo and $3.95 \pm 0.20$ before L-ASA. $p < 0.05$ compared to placebo at the same time point, paired t test.

Figure 3. Time course of sRaw changes, expressed as percentage of post-treatment value (time 0). Baseline sRaw were $0.32 \pm 0.32$ kPa.s ($M \pm SE$) before placebo and $0.35 \pm 0.01$ before L-ASA, respectively. Symbols as for figure 2.

Figure 4. Effect of inhaled L-ASA on the late asthmatic reaction to allergen challenge in a patient sensitive to pollen (predicted $FEV_1$ 3.49 l), measured as changes of $FEV_1$ (left) and sRaw (right).

Figure 5. Effect of inhaled L-ASA on the late asthmatic reaction to allergen challenge in an allergic patient (predicted $FEV_1$ 3.37 l), measured as changes of $FEV_1$ (left) and sRaw (right).

Table I

| Effect of inhaled L-ASA on UNW-induced bronchial reactions. | | | | | | |
|---|---|---|---|---|---|---|
| (Patients' characteristics) | | | | | | |
| N. | Sex | Age | $FEV_1$ | | ALLERGY[a] | THERAPY[b] |
| | | | litre | %pred | | |
| 1 | M | 34 | 3.85 | 94 | DP | B2 + CS |
| 2 | M | 24 | 3.18 | 78 | - | B2 |
| 3 | F | 39 | 2.47 | 98 | - | B2 + CS |
| 4 | F | 19 | 3.08 | 100 | GR | B2 |
| 5 | F | 35 | 2.10 | 75 | - | B2 + CS |
| 6 | F | 40 | 2.32 | 90 | GR | B2 |
| 7 | M | 18 | 3.29 | 81 | - | B2 |

a) DP: Dermatophagoides, GR: Grass pollen

b) B2: inhaled beta2-agonist, CS: inhaled steroids.

## Claims

1.  Use of the non-steroidal anti-inflammatory drug acetylsalicylic acid for the preparation of a medicament for the inhalation therapy of asthma.

## Patentansprüche

1.  Gebrauch des antiinflammatorischen nicht-stereoidischen Medikamentes Acetylsalicylsäure für die Zubereitung eines Arzneimittels für die Inhalationstherapie von Asthma.

## Revendications

1.  Usage du médicament acide acetylsalicylique non-steroidique anti-inflammatoire pour la préparation d'un médicament pour la thérapie inhalative du asthme.

*Fig. 1*

*Fig. 2*

FEV₁ CHANGES [%]

PRE - TREAT

●—● L - ASA
○—○ Placebo

TIME AFTER CHALLENGE [minutes]

*Fig. 3*

♀ 24yr. - PARIETARIA POLLEN

Placebo
L-ASA

TIME AFTER CHALLENGE [hours]

Fig. 4

EP 0 499 143 B1

♀ 19 yr. – DERMATOPHAGOIDES

Fig. 5

Placebo
L-ASA

TIME AFTER CHALLENGE [hours]

EP 0 499 143 B1